# EUROPEAN PATENT SPECIFICATION

(11) **EP 0 470 977 B1**
(45) Date of publication and mention of the grant of the patent: **28.08.1996**
(21) Application number: 90906745.6
(22) Date of filing: 01.05.1990
(51) Int. Cl.: A61M 5/50, A61M 5/315

(54) **A SYRINGE**
SPRITZE
SERINGUE

(30) Priority: 04.05.1989 AU PJ4034/89
(43) Date of publication of application: 19.02.1992
(73) Proprietor: WESTERN MEDICAL PRODUCTS PTY LIMITED, Sydney, NSW 2000 (AU)
(72) Inventor: WHITLEY, Kevin, Redfern, NSW 2016 (AU)
(74) Representative: Bannerman, David Gardner
(86) International application number: AU9000175
(87) International publication number: WO9013325

(56) References cited:
- EP-A- 0 307 367
- GB-A- 1 382 941
- US-A- 2 626 604
- US-A- 3 306 290
- US-A- 3 890 971
- US-A- 4 747 829
- US-A- 4 770 655
- US-A- 4 834 718

## Description

The present invention relates to syringes and, in particular, to a syringe which is able to be disarmed after use.

### BACKGROUND ART

It is the modern practice in medicine to dispose of used syringes and needles after only one operation. This practice has arisen due to possible contamination of the needle and syringe when administering to a patient.

Such a practice is strictly adhered to by medical practitioners and other ancillary staff in medical institutions. Therefore, the main problem in this regard in relation to hospitals and the like is not whether the syringes are used more than once but whether the syringes are disposed of correctly to avoid accidental skin puncture known as "needle stick". In this case responsible users of the syringes ensure that the syringes are correctly disposed of.

With the advent of increased use of intravenous drugs by drug addicts, there is an increasing incidence of repeated use of syringes by such users, and even several drug users using the same needle. This is unhygienic and tends to lead to the spread of infectious diseases, in particular hepatitis and AIDS.

It has been noted that education and free distribution of syringes and needles is one way of overcoming the possibility of the spread of such infectious diseases. The needle exchange programmes which have been initiated require that a syringe be returned before another one is issued. However, it has been acknowledged that these schemes are only successful up to a point, as it is possible for most conventional syringes to be used more than once, and used for more than one person. This ability to be re-used increases the risk of the spread of such diseases.

EP 0307367A discloses a once only syringe comprising a needle protection cap. The syringe comprising elastic means which are initially located in an inactive position and are transferred into an active position during a first syringe intake operation. When in said active position the elastic means are disposed in a position secured to the body of the syringe, and prevent the syringe being reused. The syringe also comprises a slidable cap coaxial with the syringe body for covering the needle.

US 3890971 discloses a one-time use safety syringe comprising a plunger which is permanently lockable by detent members and a slidable needle cap which is also permanently lockable by detents to prevent reuse.

US 4834718 discloses a clinical needle with a protecting sheath which is moved over the needle when the needle is withdrawn from the patient. This shields the needle so preventing needle stick injuries.

US 4770655 discloses a syringe in which the needle is withdrawn into a cylinder. A formation at the distal end of the cylinder is used to destroy the needle.

US 4747829 discloses a syringe with a retractable needle. Means are provided on the barrel of the syringe to block the needle after it has been retracted with the barrel, thus preventing the re-use of the syringe.

### OBJECT OF THE INVENTION

It is the primary object of the present invention to provide a syringe which is able to be disarmed after use to thereby prevent "needle stick". It is a preferred feature of the present invention to provide such a syringe which is only able to be used once.

### DISCLOSURE OF THE INVENTION

According to one aspect of the present invention there is disclosed a syringe to prevent "needle stick" after use, said syringe 1 comprising an inner elongate sheath 4 slidably located within an outer elongate sheath 3, said inner sheath 4 having at one end thereof needle means communicating with the interior of said inner sheath and passing through and beyond the corresponding end of said outer sheath 3 when said inner sheath 4 is closely adjacent said outer sheath corresponding end 5, a plunger 9 slidably mounted in said inner sheath 4 interior from the other end thereof and carrying a piston 10, said piston 10 and said inner sheath 4 interior defining a liquid drug receiving cavity of adjustable volume, and comprising a first 13, 18 and second locking means 22, 26; the outer elongate sheath 3 forms the body of the syringe when held by the operator during the administration of an injection, and the first two part locking means comprises one part 13 which is carried by said plunger 9 and another part which is carried by said inner sheath 18, said first two part locking means being positioned on said plunger 9 and inner sheath 4 at respected locations to inter-engage said two parts when said drug receiving cavity is substantially reduced to its minimum working volume by movement of said plunger 9 towards said needle means 7 to thereby cause said plunger and inner sheath to move together relative to said outer sheath, and a second two-part locking means one part 22 of which is carried by said outer sheath 3 and the other part 26 of which is carried by said inner sheath 4, said second two part locking means 22, 26 being arranged to inter-engage when said inner sheath 4 is moved relative to said other sheath 3 to draw said needle means into said outer sheath to thereby prevent said inner 4 and outer 3 sheaths sliding longitudinally relative to each other, and the length of said needle means 7 and said outer sheath 3 being selected to fully locate said needle means 7 within said outer sheath 3 when said second locking means 19, 22 is inter-engaged.

### BRIEF DESCRIPTION OF THE DRAWINGS

One embodiment of the present invention will now be described with reference to the drawings in which:

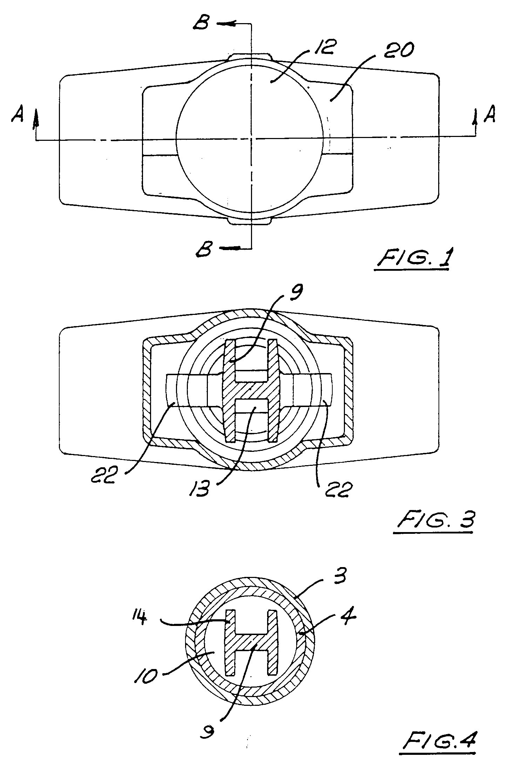

Fig. 1 is an end elevation of the syringe of the preferred embodiment,
Fig. 2 is a longitudinal sectional view along the line A-A of Fig. 1,
Fig. 3 is a transverse sectional view along the line C-C of Fig. 2,
Fig. 4 is a transverse sectional view along the line D-D of Fig. 2,
Fig. 5 is a longitudinal sectional view along the line B-B of Fig. 1,
Fig. 6 is a longitudinal sectional view along the line A-B of Fig. 1, and
Fig. 7 is a perspective view of the syringe of the preferred embodiment.

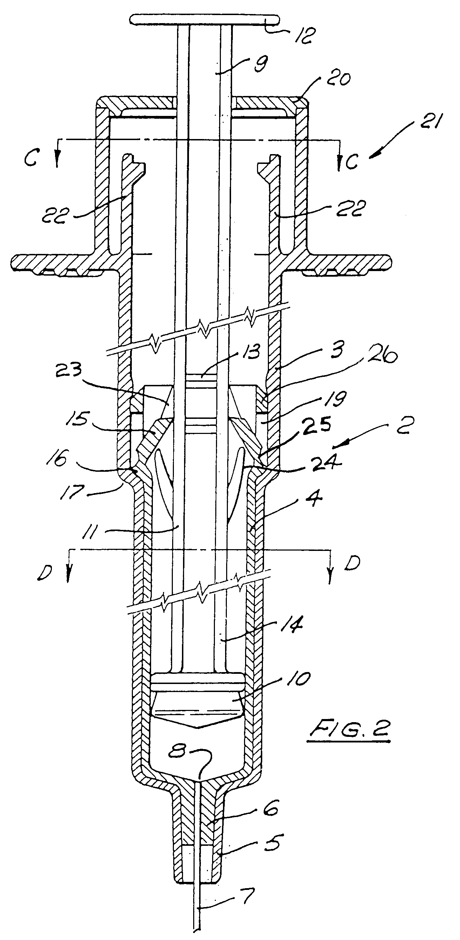

As best seen in Fig. 7, the syringe 1 of the preferred embodiment has a barrel 2 having an outer sheath 3 and an inner sheath 4 (Fig. 2). The outer sheath 3 has a nozzle 5 and likewise the inner sheath 4 has a nozzle 6 the exterior of which is a friction fit within the interior of the nozzle 5. A needle 7 also is a friction fit within a central aperture 8 in the nozzle 6 of the inner sheath 4.

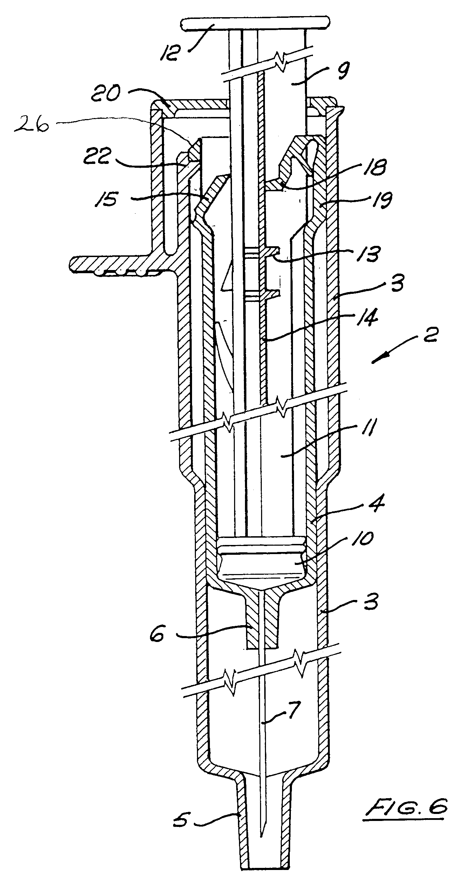

A plunger 9 is generally similar to the plunger of a standard conventional syringe, as it includes a piston 10, a stem 11 and a base 12. However, a pair of locking ribs 13 (Figs. 2 and 5) are located approximately midway on the stem 11 between two of the four flange portions 14 (Fig. 4) of the stem 11. A pair of safety locks 15 are integrally formed on the inner sheath 4. A pair of stops 23 on the plunger 9 are located adjacent the locking ribs 13 on the stem 11 and together with the locks 15 prevent accidental plunger lock before use. The safety locks 15 are connected to the inner sheath 4 by a thin piece of plastics material 25 which is able to be deformed.

The inner sheath 4 prior to use is as illustrated in Fig. 2 and fits snugly within the outer sheath 3. The inner sheath 4 has a shoulder 16 which fits within a corresponding shoulder 17 of the outer sheath 3. The location of the shoulders 16 and 17 is approximately midway along the barrel 2. The pair of safety locks 15 are seen in Fig. 2 to be resting against the pair of stops 23 so that the plunger 9 is not able to be moved in a downwards direction. Thus the safety locks 15 and stops 23 prevent accidental downwards movement of the plunger prior to use which could lock mechanism of syringe 1 in a manner to be described hereafter.

A pair of resilient protruding fingers 24 are also located on the stem 11 of the plunger 9 and are arranged to interact with the safety locks 15 as the plunger 9 is withdrawn towards the needle 7 through a short distance.

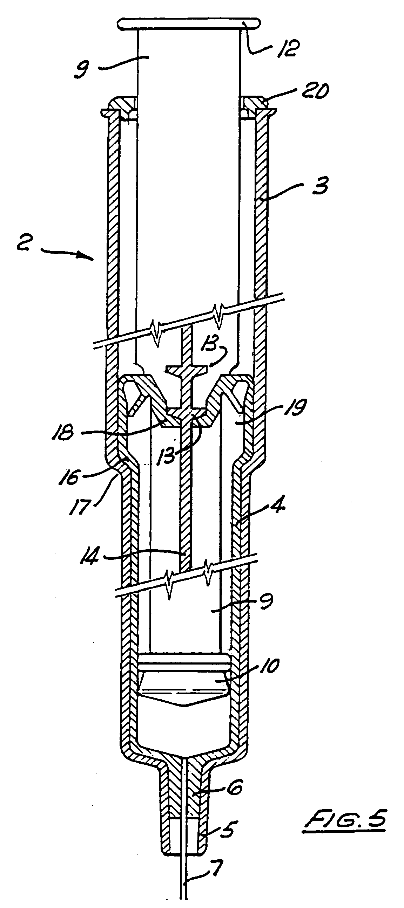

As illustrated in Fig. 3, a pair of plunger locks 18 are located at the upper end 19 of the inner sheath 4. The plunger locks 18 are provided to interact with the locking ribs 13 after a single use of the syringe 1. Both the plunger locks 18 and the locking ribs 13 are made of resilient material.

A sealing cap 20 is located at the upper end 21 of the outer sheath 3 of the barrel 2. The sealing cap 20 is formed in two portions each integrally moulded with the barrel 3 of the syringe 1 and hinged thereto. After assembly of the syringe these two portions are welded to the barrel 3. This effectively seals the outer sheath 3.

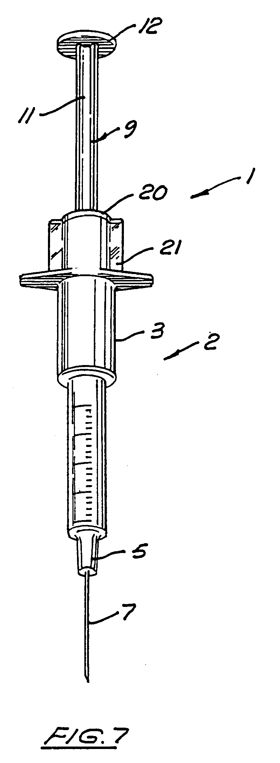

A pair of locking hooks 22 (Figs. 2 and 6) are located within the outer sheath 3 adjacent the sealing cap 20. The locking hooks 22 interact with corresponding protrusions 26 on the upper end 19 of the inner sheath 4 to prevent movement therebetween.

The syringe 1 is illustrated as ready for initial charging in Fig. 2. In this initial configuration, the inner sheath 4 and outer sheath 3 are in a position where the needle 7 is exposed, and the plunger 9 is ready to draw in a drug. First the plunger 9 is drawn in an upwards direction thus drawing in the liquid drug into the inner sheath 4 via the needle 7. This is a substantially conventional operation.

As the plunger 9 is drawn in the upwards direction, the fingers 24 deflect the safety locks 15 thus elastically deforming the thin piece of plastics material 25 which joins the safety locks 15 to the inner sheath 4. After this initial action, the safety locks 15 lie closer to the outer sheath 3 than the position illustrated in Fig. 2. Therefore the locks 15 are not able to prevent the downward movement of the plunger 9 by interaction with the stops 23 after the initial charging.

Due to the initial air gap between the piston 10 and the nozzle 6 as seen in Fig. 2, a small amount of air is mixed with the drug when drawn into the barrel 2. As is usual practice, this air is expelled prior to injection by moving the plunger 9 towards the needle 7 with the syringe held with the needle 7 uppermost.

Once the syringe 1 is thus readied for use, the plunger 9 is pressed in a downwards (with reference to the orientation of the syringe illustrated in Figs. 2, 5 and 6) motion thus injecting the drug through the needle 7 by the piston 10 expelling the drug from the inner sheath 4. As illustrated in Fig. 5, shortly before the piston 10 reaches the end of its maximum distance of travel, the lower one of the two locking ribs 13 abuts the plunger locks 18. In this position the plunger 9 has reached what may be termed "the greed factor point". During unauthorized use of the syringe, particularly with initially gratifying illegal drugs, it is envisaged that the plunger 9 will be driven fully home so as to inject every possible drop of the drug.

This final motion of the plunger 9 moves the locking ribs 13 beyond the plunger locks 18. As a consequence, the inner sheath 4 and plunger 9 are locked together within the outer sheath 3 and an unobtrusive manner.

It will be appreciated that prior to engagement of the locking ribs 13 and plunger locks 18 the plunger 9 can be reciprocated within the inner sheath 4. This can be of assistance in expelling air from the drug or even re-filling the syringe if a greater volume of drug is to be administered than can be stored within the inner sheath 4.

Once the locking ribs 13 and plunger locks 18 have locked together, when the plunger 9 is pulled in a upward motion, the inner sheath 4 is also pulled upwardly. Thus the needle 7 is retracted into the interior of the outer sheath 3 as seen in Fig. 6. The syringe 1 is now able to be disposed of safely due to the covering of the needle 7 by the outer sheath 3. This helps to prevent "needle stick". The syringe 1 is also disarmed by the locking of the plunger 9 within the inner sheath 4 so that it cannot be used again.

However, it is desirable that the needle 7 be locked in the retracted position as illustrated in Fig. 6. Sufficient upward movement of the plunger 9, moves the protrusions 26 past the locking hooks 22 as seen in Fig. 6. This locks the outer sheath 3 and inner sheath 4 thus preventing any movement of the inner sheath 4 within the outer sheath 3. The syringe as well as being locked in a disarmed condition is also locked in a safe condition and is thus able to be disposed of safely.

The foregoing describes only one embodiment of the present invention, and modifications obvious to those skilled in the art can be made thereto without departing from the scope of the present invention.

## Claims

1. A syringe to prevent "needle stick" after use, said syringe (1) comprising an inner elongate sheath (4) slidably located within an outer elongate sheath (3), said inner sheath (4) having at one end thereof needle means (7) communicating with the interior of said inner sheath and passing through and beyond the corresponding end of said outer sheath (3) when said inner sheath (4) is closely adjacent said outer sheath corresponding end (5), a plunger (9) slidably mounted in said inner sheath (2) interior from the other end thereof and carrying a piston (10), said piston (10) and said inner sheath (4) interior defining a liquid drug receiving cavity of adjustable volume, and comprising a first (13, 18) and second locking means (22, 26); characterised in that the outer elongate sheath (3) forms the body of the syringe when held by the operator during the administration of an injection, and the first two part locking means comprises one part (13) which is carried by said plunger (9) and another part which is carried by said inner sheath (18), said first two part locking means being positioned on said plunger (9) and inner sheath (4) at respected locations to inter-engage said two parts when said drug receiving cavity is substantially reduced to its minimum working volume by movement of said plunger (9) towards said needle means (7) to thereby cause said plunger and inner sheath to move together relative to said outer sheath, and a second two-part locking means one part (22) of which is carried by said outer sheath (3) and the other part (20) of which is carried by said inner sheath (4), said second two-part locking means (22, 26) being arranged to inter-engage when said inner sheath (4) is moved relative to said other sheath (3) to draw said needle means into said outer sheath to thereby prevent said inner (4) and outer (3) sheaths sliding longitudinally relative to each other, and the length of said needle means (7) and said outer sheath (3) being selected to fully locate said needle means (7) within said outer sheath (3) when said second locking means (19, 22) is inter-engaged.

2. A syringe as claimed in Claim 1, wherein said two parts of said second locking means (22, 26) are positioned on said outer (3) and inner (4) sheaths to permit said inter-engagement thereof only when said needle means (7) of said inner sheath (4) is withdrawn into said outer sheath (3) to substantially a maximum extent.

3. A syringe as claimed in Claim 1, wherein said second locking means (22, 26) is not able to be released.

4. A syringe as claimed in Claim 3, wherein said plunger (9) extends through one end of said outer sheath (3) through a cap means (30) sealing said one end of said outer sheath (3), said cap means shielding said second locking means (22, 26).

5. A syringe as claimed in any one of Claims 1 to 4, wherein said first locking means (13, 18) is not releasable to prevent said syringe being re-used after its internal use.

6. A syringe as claimed in any one of Claims 1 to 5, wherein said plunger (9) is slidably reciprocable within said inner sheath (4) prior to reducing said drug receiving cavity to said minimum working volume.

7. A syringe as claimed in any one of Claims 1 to 6, hwerien a two-part deformable stop means (15, 24, 25) is provided, one part (25) of which is deformable and located on said inner sheath (4) and the other part (24) of which is located on said plunger (9), said stop means (15, 24, 25) parts inter-engaging to prevent motion of said plunger (9) towards said needle means to an extent sufficient to inter-engage said first locking means (13, 18), and said stop means (15, 25) being deformable by deflection means (24) located on said plunger and movable past said one stop means (15) past during initial movement of said plunger (9) to fill said drug receiving cavity, after said deformation said one stop means part (15) not being engageable with said other stop means part (24).

8. A syringe as claimed in any one of Claims 1 to 7, wherein said inner (4) and outer (3) sheaths are shaped to provide a stop abutment (16, 17) therebetween to limit the penetration of said inner sheath (4) into said outer sheath (3).

9. A syringe as claimed in Claim 8, wherein said stop abutment takes the form of substantially identical profiles on the exterior of said inner sheath (16) and the interior of said outer sheath (17), said profiles providing a friction fit when abutted.

## Patentansprüche

1. Eine Spritze (1) zum Vermeiden von "Nadelstechen" nach dem Gebrauch umfaßt
eine innere verlängerte Hülse (4), die verschiebbar in einer äußeren verlängerten Hülse (3) angeordnet ist, wobei die innere Hülse (4) an einem Ende eine Nadel (7) aufweist, die mit dem Inneren der inneren Hülse kommuniziert und durch und über das entsprechende Ende der äußeren Hülse (3) hinausragt, wenn die innere Hülse (4) eng an das korrespondierende Ende (5) der äußeren Hülse angrenzt,
einen Kolben (9), der innerhalb der inneren Hülse (2) von ihrem anderen Ende aus verschiebbar angeordnet ist und mit einem Stempel (10) versehen ist, wobei der Stempel (10) und die innere Hülse (4) im Inneren einen Hohlraum mit veränderbarem Volumen zur Aufnahme eines flüssigen Arzneimittels definieren,
und umfaßt erste (13, 18) und zweite Verschlußmittel (22, 26);
**dadurch gekennzeichnet**, daß die äußere verlängerte Hülse (3) den Körper der Spritze bildet, der von der Bedienungsperson während der Verabreichung einer Injektion gehalten wird,
und daß das erste zweiteilige Verschlußmittel einen Teil (13) umfaßt, der von dem Kolben (9) getragen wird, und ein weiteres Teil, das von der inneren Hülse (18) getragen wird, wobei das erste zweiteilige Verschlußmittel auf dem Kolben (9) und der inneren Hülse (4) derart angeordnet ist, daß die zwei Teile ineinandergreifen, wenn der Hohlraum für flüssiges Arzneimittel durch die Bewegung des Kolbens (9) gegen die Nadel (7) im wesentlichen auf sein minimales Arbeitsvolumen reduziert ist, um dabei den Kolben und die innere Hülse zusammen relativ zu der äußeren Hülse zu bewegen,
und daß ein zweites zweiteiliges Verschlußmittel vorgesehen ist, wobei ein Teil (22) von der äußeren Hülse (3) getragen wird und das andere Teil (20) von der inneren Hülse (4) getragen wird, wobei das zweite zweiteilige Verschlußmittel (22, 26) so angeordnet ist, daß es ineinandergreift, wenn die innere Hülse (4) relativ zur äußeren Hülse (3) bewegt wird, um die Nadel in die äußere Hüls zu ziehen und dabei zu verhindern, daß sich die innere (4) und die äußere Hülse (3) longitudinal realtiv zueinander verschieben,
und daß die Länge der Nadel (7) und der äußeren Hülse (3) so gewählt ist, daß die Nadel (3) vollständig in der äußeren Hülse (3) angeordnet wird, wenn die zweiten Verschlußmittel (19, 22) ineinandergreifen.

2. Eine Spritze nach Anspruch 1, wobei die zwei Teile der zweiten Verschlußmittel (22, 26) in der äußeren (3) und inneren (4) Hülse angeordnet sind, um ein Ineinandergreifen nur zu ermöglichen, wenn die Nadel (7) der inneren Hülse (4) im wesentlichen maximal in die äußere Hülse (3) zurückgezogen wird.

3. Eine Spritze nach Anspruch 1, wobei die zweiten Verschlußmittel (22, 26) nicht gelöst werden können.

4. Eine Spritze nach Anspruch 3, wobei sich der Kolben (9) durch ein Ende der äußeren Hülse (3) durch eine Kappe (30), die das eine Ende der äußeren Hülse (3) abdichtet, erstreckt, wobei die Kappe die zweiten Verschlußmittel (22, 26) abschirmt.

5. Eine Spritze nach einem der Ansprüche 1 bis 4, wobei die ersten Verschlußmittel (13, 18) nicht lösbar sind, um eine Wiederverwendung der Spritze nach ihrer inneren Verwendung zu verhindern.

6. Eine Spritze nach einem der Ansprüche 1 bis 5, wobei der Kolben (9) vor dem Reduzieren des Hohlraums für Arzneimittel auf das minimale Arbeitsvolumen reziprok innerhalb der inneren Hülse (4) verschiebbar ist.

7. Eine Spritze nach einem der Ansprüche 1 bis 6, wobei ein zweiteiliges deformierbares Stoppmittel (15, 24, 25) vorgesehen ist, von dem ein Teil (25) deformierbar ist und auf der inneren Hülse (4) angeordnet ist, und das andere Teil (24) auf dem Kolben (9) angeordnet ist, wobei die Stoppmittel (15, 24, 25) Teile ineinandergreifen, um eine Bewegung des Kolbens (9) in Richtung der Nadeln in einem zum Ineinandergreifen der ersten Verschlußmittel (13, 18) hinreichenden Ausmaß zu verhindern, und wobei die Stoppmittel (15, 25) über Biegemittel (24) deformierbar sind, die auf dem Kolben angeordnet sind und über die Stoppmittel 15 bewegbar sind während der Anfangsbewegung des Kolbens (9) zum Füllen des Hohlraums für Arzneimittel, wobei nach der Deformation das Stoppmittelteil (15) nicht mit den anderen Stoppmittelteilen (24) in Eingriff bringbar ist.

8. Eine Spritze nach einem Ansprüche 1 bis 7, wobei die innere (4) und äußere (3) Hülse so geformt sind, daß ein Widerlager (16, 17) dazwischen ausgebildet ist, um das Eindringen der inneren Hülse (4) in die äußere Hülse (3) zu begrenzen.

9. Eine Spritze nach Anspruch 8, wobei das Widerlager die Form im wesentlichen identischer Profile auf der Außenseite der inneren Hüle (16) und dem inneren der äußeren Hülse (17) aufweist, wobei die Profile eine Reibungspassung bilden, wenn sie aneinanderstoßen.

## Revendications

1. Seringue pour éviter une "piqûre d'aiguille" après utilisation, cette seringue (1) comprenant une gaine allongée intérieure (4) montée en glissement à l'intérieur d'une gaine allongée extérieure (3), la gaine intérieure (4) comportant, à une extrémité de celle-ci, une aiguille (7) communiquant avec l'intérieur de la gaine intérieure et passant à travers et au-delà de l'extrémité correspondante de la gaine extérieure (3) lorsque la gaine intérieure (4) est tout près de l'extrémité correspondante (5) de la gaine extérieure, un plongeur (9) monté en glissement à l'intérieur de la gaine intérieure (2) par l'autre extrémité de celle-ci et portant un piston (10), ce piston (10) et l'intérieur de la gaine intérieure (4) définissant une cavité de réception de médicament liquide de volume réglable, et comprenant un premier (13, 18) et un second (22, 26) moyens de verrouillage ;
caractérisée en ce que
la gaine allongée extérieure (3) forme le corps de la seringue lorsqu'elle est tenue en main par l'opérateur pendant l'administration d'une injection, et le premier moyen de verrouillage en deux parties comprend une partie (13) portée par le plongeur (9) et une autre partie portée par la gaine intérieure (18), ce premier moyen de verrouillage en deux parties étant positionné sur le plongeur (9) et la gaine intérieure (4) à des emplacements définis pour entre-engager les deux parties lorsque la cavité de réception de médicament est complètement réduite à son volume de travail minimum par le mouvement du plongeur (9) vers l'aiguille (7), afin d'amener le plongeur et la gaine intérieure à se déplacer solidairement par rapport à la gaine extérieure, et un second moyen de verrouillage en deux parties dont une partie (22) est portée par la gaine extérieure (3) et dont l'autre partie (20) est portée par la gaine intérieure (4), ce second moyen de verrouillage en deux parties (22, 26) étant disposé pour s'entre-engager lorsque la gaine intérieure (4) est déplacée par rapport à l'autre gaine (3), de manière à tirer l'aiguille dans la gaine extérieure pour empêcher ainsi la gaine intérieure (4) et la gaine extérieure (3) de glisser longitudinalement l'une par rapport à l'autre, la longueur de l'aiguille(7) et la longueur de la gaine extérieure (3) étant choisies pour loger complètement l'aiguille (7) à l'intérieur de la gaine extérieure (3) lorsque le second moyen de verrouillage (19, 22) est entre-engagé.

2. Seringue selon la revendication 1,
caractérisée en ce que
les deux parties du second moyen de verrouillage (22, 26) sont positionnées sur la gaine extérieure (3) et la gaine intérieure (4) pour ne permettre leur entre-engagement que lorsque l'aiguille (7) de la gaine intérieure (4) est retirée à l'intérieur de la gaine extérieure (3) sur une longueur essentiellement maximum.

3. Seringue selon la revendication 1,
caractérisée en ce que
le second moyen de verrouillage (22, 26) ne peut être libéré.

4. Seringue selon la revendication 3,
caractérisée en ce que
le plongeur (9) passe à travers une extrémité de la gaine extérieure (3) en traversant un capuchon (20) fermant cette extrémité de la gaine extérieure (3), ce capuchon protégeant le second moyen de verrouillage (22, 26).

5. Seringue selon l'une quelconque des revendications 1 à 4,
caractérisée en ce que
le premier moyen de verrouillage (13, 18) n'est pas libérable pour empêcher de réutiliser la seringue après son utilisation interne.

6. Seringue selon l'une quelconque des revendications 1 à 5,
caractérisée en ce que
le plongeur (9) peut glisser dans un mouvement de va-et-vient à l'intérieur de la gaine intérieure (4) avant qu'on réduise la cavité de réception de médicament à son volume de travail minimum.

7. Seringue selon l'une quelconque des revendications 1 à 6,
caractérisée en ce que
on utilise un moyen d'arrêt déformable en deux parties (15, 24, 25) dont une partie (25) est déformable et placée sur la gaine intérieure (4), tandis que son autre partie (24) est placée sur le plongeur (9), les parties de ce moyen d'arrêt (15, 24, 25) s'entre-engageant pour empêcher le mouvement du plongeur (9) vers l'aiguille avec une amplitude suffisante pour entre-engager le premier moyen de verrouillage (13, 18), et le moyen d'arrêt (15, 25) étant déformable par un moyen de déviation (24) placé sur le plongeur et pouvant se déplacer au-delà du moyen d'arrêt (15) pendant le mouvement initial du plongeur (9) pour remplir la cavité de réception de médicament, cette partie du dispositif d'arrêt (15) ne pouvant plus s'engager contre l'autre partie du dispositif d'arrêt (24) après cette déformation.

8. Seringue selon l'une quelconque des revendications 1 à 7,
caractérisée en ce que
la gaine intérieure (4) et la gaine extérieure (3) sont formées de manière à fournir une butée d'arrêt (16, 17) entre elles pour limiter la pénétration de la gaine intérieure (4) dans la gaine extérieure (3).

9. Seringue selon la revendication 8
caractérisée en ce que
la butée d'arrêt prend la forme de profils essentiellement identiques sur l'extérieur de la gaine intérieure (16) et sur l'intérieur de la gaine extérieure (17), ces profils fournissant un emboîtement à friction lorsqu'ils sont en butée.
